# FASCICULE DE BREVET EUROPEEN

(11) **EP 1 969 025 B1**
(45) Date de publication et mention de la délivrance du brevet: **21.02.2018**
(21) Numéro de dépôt: 06831730.4
(22) Date de dépôt: 13.12.2006
(51) Int. Cl.: C08G 18/28, C08G 18/48, A61K 8/87, C11D 1/72, C11D 3/37

(54) **COMPOSITIONS AQUEUSES EPAISSISSANTES ET LIMPIDES, LEUR UTILISATION DANS DES FORMULATIONS AQUEUSES ACIDES, FORMULATIONS OBTENUES**
KLARE UND VERDICKEND WIRKENDE WÄSSRIGE ZUSAMMENSETZUNGEN, VERWENDUNG DAVON IN SAUREN WÄSSRIGEN FORMULIERUNGEN UND RESULTIERENDE FORMULIERUNGEN
CLEAR AND THICKENING AQUEOUS COMPOSITIONS, THE USE THEREOF IN ACIDIC AQUEOUS FORMULATIONS, AND THE RESULTING FORMULATIONS

(30) Priorité: 20.12.2005 FR 0512929
(43) Date de publication de la demande: 17.09.2008
(73) Titulaire: COATEX, 69730 Genay (FR)
(72) Inventeur: KENSICHER, Yves, F-69620 Theize (FR)
(86) Numéro de dépôt international: PCT/IB2006/003645
(87) Numéro de publication internationale: WO 2007/072152

(56) Documents cités:
- EP-A- 0 825 229
- EP-A- 0 875 557
- EP-A- 1 120 102
- EP-A- 1 584 331
- US-A1- 2005 187 342

## Description

Le domaine technique de la présente invention est constitué des formulations aqueuses dites acides (on entend ici strictement par acide un pH inférieur à 7), telles que notamment pour la détergence, le détartrage, et les applications cosmétiques où un pH inférieur à 7 est requis.

Un premier objet de l'invention consiste en des compositions aqueuses épaississantes et limpides contenant :
(a) de 10 à 80 % d'au moins 2 tensioactifs non ioniques, étant des alcools gras oxyalkylés,
(b) de 4 à 40 % d'au moins 1 composé polyuréthanne, constitué d'au moins un groupement du type alkyle ou aryle ou aryalkyle ou le mélange de ces groupements, d'au moins un groupement du type polyalkylène glycol qui est préférentiellement le polyéthylène glycol, et d'au moins un groupement isocyanate particulier tel qu'explicité plus loin dans la présente Demande,
(c) et de l'eau.

Un autre objet de l'invention est l'utilisation de telles compositions en vue d'épaissir des formulations aqueuses acides tout en maintenant leur caractère limpide.

Un dernier objet de l'invention réside dans les formulations aqueuses acides limpides et ainsi épaissies.

Dans le secteur des formulations aqueuses, il est un besoin bien connu d'épaissir lesdites formulations (c'est-à-dire d'augmenter leur viscosité) pour leur bonne applicabilité lorsqu'elles sont mises en oeuvre par l'utilisateur final.

Pour ce faire, l'homme du métier (formulateur de telles compositions) développe depuis de nombreuses années des additifs modificateurs de rhéologie, dont le principal effet est celui d'épaissir les formulations qu'il fabrique ; ces additifs portent le nom d'épaississants, qu'ils soient d'origine naturelle ou synthétique.
Dans le domaine plus restreint qui intéresse la présente invention c'est-à-dire des formulations aqueuses dites acides (pH < 7), l'emploi d'épaississants acryliques classiques est bien évidemment impossible car on sait que ceux-ci s'avèrent peu efficaces à de tels pH.
On a donc recours à d'autres types d'épaississants, dont notamment les polyuréthanes, mais il en existe toutefois d'autres.

Ainsi, l'homme du métier connaît les épaississants à base de xanthane, relativement efficaces en terme de pouvoir épaississant, mais qui posent néanmoins deux problèmes majeurs.

Le premier est qu'il se présentent sous forme de poudre sèche, ladite poudre devant préalablement être dispersée en milieu aqueux, ce qui constitue une étape de mise en oeuvre supplémentaire, donc un surcoût et des investissements supplémentaires, outre le fait de choisir pour l'homme du métier ou l'utilisateur final le dispersant ad hoc pour réaliser cette dispersion.

Le second est que, pour des applications ménagères, telle que le détartrage d'appareillages domestiques, il est un argument rédhibitoire à l'encontre de ce type d'épaississants : en milieu aqueux, ils donnent lieu à une solution trouble qui est aux antipodes de l'attente de l'utilisateur final, en terme de présentation et de "packaging" (conditionnement).

L'homme du métier connaît également l'utilisation de mélanges du type amines quaternaires avec différents tensioactifs. Il faut cependant souligner que leur efficacité repose sur une mise au point - souvent difficile - du couple amine / tensioactif. Mais le principal argument à leur encontre est d'ordre environnemental : leur nature cationique les prédispose à s'adsorber sur les corps anioniques (telles que notamment les branchies des poissons), ce qui fait d'eux des dangers mortels pour la faune aquatique.
Par conséquent, l'homme du métier se tourne résolument vers les épaississants de type polyuréthannes.

Afin d'être mis en oeuvre, de tels épaississants de type polyuréthanne doivent être solubilisés ; l'homme du métier a alors recours à des solutions qui consistent en la mise en oeuvre d'additifs souvent dénommés sous l'expression de tensioactifs, ou encore de surfactants. La Demanderesse indique qu'alors, les compositions ainsi formulées peuvent conduire à des problèmes de limpidité, comme dans le cas des composés xanthanes.
A ce titre, l'homme du métier connaît le document EP 1 093 488, qui concerne le problème technique de produire des polymères associatifs épaississants fortement concentrés sans employer de solvants et en employant une faible quantité de modificateurs de rhéologie. Ce document décrit l'utilisation d'un tensioactif non ionique de formule RO-(-AO-)ₐ-H, où R désigne un groupe alkyle ayant de 6 à 12 atomes de carbone et AO désigne un groupe oxyéthylène ou oxypropylène (dans un rapport entre le nombre de groupes oxyéthylène:oxypropylène compris entre 1:2 et 5:6) avec a compris entre 7 et 16, comme agent fortement réducteur de viscosité d'un concentré aqueux d'un polymère épaississant associatif, de type polyuréthanne. Il décrit également un concentré aqueux ayant de 10 % à 50 % en poids de polyuréthanne et 1 % à 25 % en poids de tensioactif. Ces formulations sont particulièrement bien adaptées dans des applications de type peinture et vernis contenant des latex.

L'homme du métier connaît aussi le document EP 0 839 877, qui vise à résoudre le problème de la préparation d'épaississants qui présentent d'une part une viscosité abaissée mais d'autre part présentent des propriétés rhéologiques améliorées. Ce document décrit une préparation d'épaississant pour le réglage de la rhéologie de systèmes aqueux composée d'épaississants à base d'uréthane, de surfactant non ionique et d'un troisième additif (diester).
Il connaît aussi le document EP 0 682 094 dont l'objet consiste en l'élimination de la phase solvant qui permet de formuler le polyuréthanne sous forme liquide et utilisable. Il décrit dans ce but une composition épaississante associative pour le contrôle de rhéologie de systèmes aqueux, cette composition épaississante étant de viscosité inférieure à 15 000 mPa.s et étant composée, en pourcentage en poids de chaque constituant : de 15 % à 40 % d'un polymère épaississant (tel qu'un polyuréthanne), d'au moins 30 % d'eau et de 1 % à 30 % d'un ou plusieurs surfactant anioniques et / ou non ioniques.

Il connaît également le document EP 0 835 291 qui concerne la préparation d'une base polyuréthanne sous forme liquide pour préparer une formulation aqueuse d'épaississant hydrosoluble (comprenant des groupes uréthanes) en employant un agent abaissant la viscosité, ledit agent étant un surfactant contenant des unités oxyalkylène possédant de 2 à 4 atomes de carbone et un radical hydrocarboné. Ce document décrit des formulations constituées, exprimé en pourcentage en poids de chaque constituant, de 1 % à 50 % de surfactant, de 10 % à 99 % d'épaississant, et le reste d'eau.
Aucun de ces documents ne concerne le problème technique de l'invention, c'est-à-dire la préparation d'épaississants spécifiquement pour des formulations acides.

Enfin, l'homme du métier connaît le document EP 0 875 557 qui décrit une composition aqueuse d'un épaississant associatif type polyétheruréthane formulé avec un mélange d'au moins 2 tensioactifs ioniques ou non ioniques de HLB (balance lipophile - hydrophile) différentes (condition sur la valeur de la HLB et du rapport massique entre les tensioactifs du mélange) pour améliorer l'épaississement des formulations contenant cette composition, tout en diminuant la concentration en polyuréthanne. Les applications visées sont extrêmement larges, et la très grande majorité ne concernent pas le traitement de systèmes acides (page 2 lignes 50-56) sauf une brève mention d'une formulation acide pour l'hygiène (page 2 ligne 55). Il est indiqué qu'une telle composition peut néanmoins être employée dans une formulation acide contenant de 5 % à 10 % en poids d'acide phosphorique, sulfurique ou citrique et ce, de manière très générale (page 13, lignes 46-49).

Ce document n'est donc pas spécialement adapté au traitement de formulations acides, et il n'existe pas de raison objective pour que l'homme de métier soit amené à remarquer et isoler la formulation acide pour l'hygiène au milieu d'une douzaine d'autres applications non acides, comme les fluides de forage, les peintures, revêtements, et diverses lotions pour shampoing et analogues, ou pour l'agriculture et les formulations de pesticides.

Ainsi, en vue de résoudre le problème technique général d'augmenter la viscosité de formulations aqueuses acides, tout en évitant les inconvénients liés à l'utilisation de xanthanes (poudres sèches qu'il faut d'abord solubiliser, et qui conduisent ensuite à des formulations troubles) et d'amines quaternaires (produits dangereux pour la faune aquatique), l'inventeur a mis en oeuvre de manière surprenante de nouvelles compositions épaississantes et limpides contenant :
(a) de 10 à 80 % d'au moins 2 tensioactifs non ioniques étant des alcools gras oxyalkylés,
(b) de 4 à 40 % d'au moins 1 composé polyuréthanne, obtenu à partir
   - d'au moins un alcool gras,
   - d'au moins un polyalkylène glycol, préférentiellement le polyéthylène glycol, et
   - d'au moins un isocyanate choisi dans le groupe consistant en le 1,4-butane di-isocyanate, le 1,6-hexane diisocyanate, l'isophorone diisocyanate, le 1,3-cyclohexane diisocyanate, le 1,4-cyclohexane diisocyanate, le 4,4'diisocyanatodicyclohexylmethane, le 1-methyl-2,4-diisocyanatocyclohexane et son mélange avec le 1-methyl-2,6-diisocyanatocyclohexane, et leurs mélanges,
(c) et de l'eau.

De manière tout à fait surprenante, ces compositions s'avèrent tout à fait limpides, et permettent de réaliser des formulations aqueuses acides épaissies qui sont elles aussi parfaitement limpides, ce qui n'était pas le cas de toutes les compositions polyuréthannes de l'art antérieur.

De plus, de telles compositions permettent d'épaissir de manière très importante des formulations aqueuses acides les contenant et ce, de façon plus efficace que les compositions polyuréthannes de l'art antérieur.

Pour résoudre le problème général de l'augmentation de la viscosité tout en conservant un caractère limpide aussi bien à la composition épaississante qu'à la formulation aqueuse acide épaissie, le Demandeur a su remarquer que pour atteindre son objectif il convenait de très bien solubiliser le composé polyuréthanne.
Le mérite du Demandeur repose notamment sur la mise au point de mélanges d'au moins 2 tensioactifs et d'au moins un composé polyuréthanne particulier tel que décrit plus avant dans la présente Demande. Ici, les couples tensioactifs / polyuréthanne sont tels que les tensioactifs solubilisent parfaitement dans l'eau ces polyuréthannes ce qui, selon les études du demandeur, permet d'obtenir de manière surprenante un produit très efficace en terme d'effet épaississant tout en conservant un caractère limpide à la composition épaississante et à la formulation aqueuse acide épaissie.

Par conséquence, on ne produit pas de solutions troubles (comme avec les xanthanes et certains mélanges polyuréthannes de l'art antérieur), il n'existe pas de dangers vis-à-vis de l'environnement (contrairement aux amines quaternaires ou aux solvants), et la technique est simple à mettre en oeuvre (pas de problèmes de poudres comme avec les xanthanes). De plus, l'effet épaississant obtenu est très important.

Aussi, un premier objet de l'invention consiste en des compositions aqueuses épaississantes et limpides contenant :
(a) de 10 à 80 % d'au moins 2 tensioactifs non ioniques étant des alcools gras oxyalkylés,
(b) de 4 à 40 % d'au moins 1 composé polyuréthanne, obtenu à partir
   - d'au moins un alcool gras,
   - d'au moins un polyalkylène glycol, préférentiellement le polyéthylène glycol, et
   - d'au moins un isocyanate choisi parmi le 1,4-butane di-isocyanate, le 1,6-hexane diisocyanate, l'isophorone diisocyanate, le 1,3- cyclohexane diisocyanate et le 1,4- cyclohexane diisocyanate, le 4,4'diisocyanatodicyclohexylmethane, le 1-methyl-2,4-diisocyanatocyclohexane et son mélange avec le 1-methyl-2,6-diisocyanatocyclohexane, et leurs mélanges,
(c) et de l'eau.

Ces compositions aqueuses épaississantes et limpides sont aussi caractérisées en ce qu'elles contiennent, exprimé en pourcentage en poids de chaque constituant, par rapport au poids total de la formulation :
(a) préférentiellement de 20 % à 65 % d'au moins 2 tensioactifs non ioniques, étant des alcools gras oxyalkylés,
(b) préférentiellement de 10 % à 30 % d'au moins 1 composé polyuréthanne,
(c) et le reste d'eau.

Ces compositions aqueuses épaississantes et limpides sont aussi caractérisées en ce que, pour les tensioactifs qu'elles mettent en oeuvre, lesdits tensioactifs sont des alcools gras oxyalkylés non-ioniques, ayant préférentiellement de 3 à 40 motifs d'oxyéthylène, et ayant préférentiellement de 8 à 30 atomes de carbone, très préférentiellement de 8 à 20 atomes de carbone.

Ces compositions aqueuses épaississantes et limpides sont aussi caractérisées en ce que, pour les polyuréthannes qu'elles mettent en oeuvre, les groupements du type alkyle ou aryle ou aryalkyle ou le mélange de ces groupements, sont des alcools gras ayant préférentiellement de 8 à 20 atomes de carbone, très préférentiellement de 10 à 18 atomes de carbone, et extrêmement préférentiellement de 10 à 16 atomes de carbone.

Ces compositions aqueuses épaississantes et limpides sont aussi caractérisées en ce que, pour les polyuréthannes qu'elles mettent en oeuvre, les groupements polyalkylène glycol ont un poids moléculaire compris entre 5 000 g/mole et 20 000 g/mole, préférentiellement compris entre 8 000 g/mole et 15 000 g/mole, et très préférentiellement compris entre 8 000 g/mole et 12 000 g/mole.

La Demanderesse indique que dans la présente Demande, le poids moléculaire des polymères mis en oeuvre est déterminé selon la méthode GPC (Chromatographie en Phase Gel ou Gel Permeability Chromatography) mettant en oeuvre un appareil de chromatographie liquide de marque Waters™ doté de deux détecteurs dont l'un combinant la diffusion dynamique de la lumière à la viscosimétrie mesurée par un viscosimètre Viscotek™ et l'autre étant un détecteur de concentration réfractométrique de marque Waters™.
Cet appareillage de chromatographie liquide est doté de colonnes d'exclusion stérique convenablement choisies par l'homme du métier afin de séparer les différents poids moléculaires des polymères étudiés.
La phase liquide d'élution est une phase aqueuse.

Un deuxième objet de l'invention consiste en l'utilisation, pour épaissir des formulations aqueuses acides et pour conserver leur limpidité, de compositions aqueuses épaississantes et limpides contenant :
(a) de 10 à 80 % d'au moins 2 tensioactifs non ioniques étant des alcools gras oxyalkylés,
(b) de 4 à 40 % d'au moins 1 composé polyuréthanne, obtenu à partir
   - d'au moins un alcool gras,
   - d'au moins un polyalkylène glycol, préférentiellement le polyéthylène glycol, et
   - d'au moins un isocyanate choisi parmi le 1,4-butane di-isocyanate, le 1,6-hexane diisocyanate, l'isophorone diisocyanate, le 1,3-cyclohexane diisocyanate et le 1,4-cyclohexane diisocyanate, le 4,4'diisocyanatodicyclohexylmethane, le 1-methyl-2,4-diisocyanatocyclohexane et son mélange avec le 1-methyl-2,6-diisocyanatocyclohexane, et leurs mélanges,
(c) et de l'eau.

Cette utilisation est aussi caractérisée en ce que lesdites compositions aqueuses épaississantes et limpides contiennent, exprimé en pourcentage en poids de chaque constituant, par rapport au poids total de la formulation :
(a) préférentiellement de 20 % à 65 % d'au moins 2 tensioactifs non ioniques étant des alcools gras oxyalkylés,
(b) préférentiellement de 10 % à 30 % d'au moins 1 composé polyuréthanne, obtenu à partir
   - d'au moins un alcool gras,
   - d'au moins un polyalkylène glycol, préférentiellement le polyéthylène glycol, et
   - d'au moins un isocyanate choisi parmi le 1,4-butane di-isocyanate, le 1,6-hexane diisocyanate, l'isophorone diisocyanate, le 1,3-cyclohexane diisocyanate et le 1,4- cyclohexane diisocyanate, le 4,4'diisocyanatodicyclohexylmethane, le 1-methyl-2,4-diisocyanatocyclohexane et son mélange avec le 1-methyl-2,6-diisocyanatocyclohexane, et leurs mélanges,
(c) et le reste d'eau.

Cette utilisation est aussi caractérisée en ce que les compositions aqueuses épaississantes et limpides contiennent au moins 2 tensioactifs qui sont des alcools gras oxyalkylés non-ioniques, ayant préférentiellement de 3 à 40 motifs d'oxyéthylène, et ayant préférentiellement de 8 à 30 atomes de carbone, très préférentiellement de 8 à 20 atomes de carbone

Cette utilisation est aussi caractérisée en ce que les compositions aqueuses épaississantes et limpides contiennent au moins un composé polyuréthanne dont les groupements du type alkyle ou aryle ou aryalkyle ou le mélange de ces groupements sont préférentiellement des alcools gras ayant préférentiellement de 8 à 20 atomes de carbone, très préférentiellement de 10 à 18 atomes de carbone, et extrêmement préférentiellement de 10 à 16 atomes de carbone.

Cette utilisation est aussi caractérisée en ce que les compositions aqueuses épaississantes et limpides contiennent au moins un composé polyuréthanne dont les groupements polyalkylène glycol ont un poids moléculaire compris entre 5 000 g/mole et 20 000 g/mole, préférentiellement compris entre 8 000 g/mole et 15 000 g/mole, et très préférentiellement compris entre 8 000 g/mole et 12 000 g/mole.

Cette utilisation est aussi caractérisée en ce qu'on met en oeuvre de 2 % à 50 %, préférentiellement de 4 % à 20 % en poids de composition aqueuse épaississante et limpide par rapport au poids total de la formulation aqueuse acide à épaissir.

Cette utilisation est aussi caractérisée en ce que la composition aqueuse épaississante et limpide est mise en oeuvre dans une formulation aqueuse acide contenant au moins un composé acidifiant choisi notamment parmi les acides chlorhydrique, phosphorique, sulfurique, nitrique, sulfamique, acétique, borique, citraconique, citrique, diglycolique, éthylène diamine tétraacétique, fluorhydrique, formique, glucoheptonique, glutarique, glycolique, glyoxylique, iséthionique, itaconique, lactique, maléïque, malique, méthane sulfonique, méthylsuccinique, octylique, oxalique, peracétique, propionique, saccharique, succinique, valérique, ou parmi les acides phosphoniques tels que notamment, acide 1-hydroxyéthane 1,1-diphosphonique, amino triméthylène phosphonique, éthylène diamine tétraméthyl phosphonique, diéthylène triamine pentaméthyl phosphonique, hexaméthylène diamine tétraméthyl phosphonique, hydroxyéthylamino diméthyl phosphonique, 2-phosphonobutane-1,2,4-tricarboxylique, ou les mélanges de ces acides, ou encore les mélanges de ces acides avec l'eau oxygénée.
Toutefois, cette liste n'est pas exhaustive.

Cette utilisation est aussi caractérisée en ce que la composition aqueuse épaississante et limpide est mise en oeuvre dans une formulation aqueuse acide dont le pH est inférieur à 7, préférentiellement inférieur à 6, très préférentiellement inférieur à 5, et de manière encore plus préférentielle inférieur à 4.

Un dernier objet de l'invention réside dans les formulations aqueuses acides ainsi épaissies et dont l'aspect est limpide.

L'invention sera mieux comprise à la lecture de la description qui va suivre, et des exemples non limitatifs ci-dessous.

### EXEMPLES

### Exemple 1

Cet exemple illustre l'utilisation d'une composition aqueuse épaississante selon l'invention ou selon l'art antérieur, en vue d'épaissir une formulation aqueuse acide contenant de l'acide citrique.
Cet exemple illustre également les compositions aqueuses épaississantes selon l'invention, ainsi que les formulations aqueuses acides selon l'invention.

Pour chacun des essais n° 1 à 16, on commence par évaluer visuellement le caractère limpide ou trouble de chaque composition épaississante, et de chaque formulation aqueuse acide.

Enfin, on mesure la viscosité Brookfield™ de chaque formulation aqueuse acide, à 25°C et à 20 tours par minute avec le module n° 3 (selon la méthode bien connue de l'homme du métier).

On calcule ensuite le rapport R entre cette viscosité et le pourcentage massique de composition aqueuse épaississante introduite dans la formulation aqueuse acide à épaissir.

### Essai n° 1

Cet essai illustre l'art antérieur.

### Composition épaississante

L'essai met en oeuvre une composition épaississante constituée de, exprimé en pourcentage en poids des constituants par rapport au poids total de la composition :
- 10 % d'un épaississant polyuréthane constitué, exprimé en pourcentage en poids des différents monomères par rapport au poids total du polymère, de :
   - 87,0 % de polyéthylène glycol de poids moléculaire égal à 5 500 g/mol,
   - 6,3 % de toluène di-isocyanate,
   - 6,7 % d'un alcool linéaire ayant 12 atomes de carbone,
   - 90 % d'une solution aqueuse à 28 % en poids sec de lauryl éther sulfate.

Cette composition épaississante met donc en oeuvre un épaississant polyuréthane et un tensioactif qui ne peuvent pas figurer dans la présente invention. La composition épaississante ainsi réalisée est trouble.

### Formulation aqueuse acide

Cette composition épaississante est mise en oeuvre pour épaissir une formulation aqueuse acide, qui contient, exprimé en pourcentage en poids des constituants par rapport au poids total de la formulation :
- 3 % d'acide citrique,
- 14 % de la composition épaississante précitée,
- 83 % d'eau en complément de l'eau déjà présente dans la composition épaississante.

Le pH de la formulation aqueuse acide est inférieur à 2.

La formulation aqueuse acide ainsi réalisée s'avère trouble.

### Essai n° 2

Cet essai illustre l'art antérieur.

### Composition épaississante

L'essai met en oeuvre une composition épaississante constituée de, exprimé en pourcentage en poids des constituants par rapport au poids total de la composition :
- 10 % d'un épaississant polyuréthane constitué, exprimé en pourcentage en poids des différents monomères par rapport au poids total du polymère, de :
   - 87,0 % de polyéthylène glycol de poids moléculaire égal à 5 500 g/mol,
   - 6,3 % de toluène di-isocyanate,
   - 6,7 % d'un alcool linéaire ayant 12 atomes de carbone,
- 90 % d'un mélange de 2 alcool gras ayant 14 et 15 atomes de carbone, et comportant 8 motifs d'oxyéthylène,

Cette composition épaississante met donc en oeuvre un épaississant polyuréthane qui ne peut pas figurer dans la présente invention, et un tensioactif qui peut figurer dans la présente invention.
La composition épaississante ainsi réalisée est trouble.

### Formulation aqueuse acide

Cette composition épaississante est mise en oeuvre pour épaissir une formulation aqueuse acide, qui contient, exprimé en pourcentage en poids des constituants par rapport au poids total de la formulation :
- 3 % d'acide citrique,
- 14,2 % de la composition épaississante précitée,
- 82,8 % d'eau en complément de l'eau déjà présente dans la composition épaississante.

Le pH de la formulation aqueuse acide est inférieur à 2.

La formulation aqueuse acide ainsi réalisée s'avère trouble.

### Essai n° 3

Cet essai illustre l'art antérieur.

### Composition épaississante

L'essai met en oeuvre une composition épaississante constituée de, exprimé en pourcentage en poids des constituants par rapport au poids total de la composition :
- 10 % d'un épaississant polyuréthane constitué, exprimé en pourcentage en poids des différents monomères par rapport au poids total du polymère, de :
   - 69,4 % de polyéthylène glycol de poids moléculaire égal à 10 000 g/mol,
   - 5,3 % d'isophorone di-isocyanate,
   - 25,3 % d'un alcool linéaire ayant 12 atomes de carbone,
- 90 % d'une solution aqueuse à 28 % en poids sec de lauryl éther sulfate.

Cette composition épaississante met donc en oeuvre un épaississant polyuréthane qui peut figurer dans la présente invention, et un tensioactif qui ne peut pas figurer dans la présente invention.

La composition épaississante ainsi réalisée est trouble.

### Formulation aqueuse acide

Cette composition épaississante est mise en oeuvre pour épaissir une formulation aqueuse acide, qui contient, exprimé en pourcentage en poids des constituants par rapport au poids total de la formulation :
- 3 % d'acide citrique,
- 13,6 % de la composition épaississante précitée,
- 83,4 % d'eau en complément de l'eau déjà présente dans la composition épaississante.

Le pH de la formulation aqueuse acide est inférieur à 2.

La formulation aqueuse acide ainsi réalisée s'avère trouble.

### Essai n° 4

Cet essai illustre l'art antérieur.

### Composition épaississante

L'essai met en oeuvre une composition épaississante constituée de, exprimé en pourcentage en poids des constituants par rapport au poids total de la composition :
- 17,65 % d'un épaississant polyuréthane constitué, exprimé en pourcentage en poids des différents monomères par rapport au poids total du polymère, de :
   - 87,0 % de polyéthylène glycol de poids moléculaire égal à 5 500 g/mol,
   - 6,3 % de toluène di-isocyanate,
   - 6,7 % d'un alcool linéaire ayant 12 atomes de carbone,
- 15,90 % en poids d'eau,
- 21,99 % en poids d'un tensioactif non ionique éthoxylé qui est un mélange d'alcools gras ayant de 12 à 18 atomes de carbone sur la chaîne grasse, et comportant 7 motifs d'oxyde d'éthylène,
- 4,44 % en poids d'un tensioactif non ionique éthoxylé qui est un mélange d'alcools gras ayant de 12 à 18 atomes de carbone sur la chaîne grasse, et comportant 3 motifs d'oxyde d'éthylène,
- 40,02 % en poids d'un tensioactif non ionique éthoxylé qui est un mélange d'alcools gras ayant de 8 atomes de carbone sur la chaîne grasse, et comportant 35 motifs d'oxyde d'éthylène.

Cette composition épaississante met donc en oeuvre un épaississant polyuréthane qui ne peut pas figurer dans la présente invention, et un tensioactif qui peut figurer dans la présente invention.

La composition épaississante ainsi réalisée est trouble.

### Formulation aqueuse acide

Cette composition épaississante est mise en oeuvre pour épaissir une formulation aqueuse acide, qui contient, exprimé en pourcentage en poids des constituants par rapport au poids total de la formulation :
- 3 % d'acide citrique,
- 10,4 % de la composition épaississante précitée,
- 86,6 % d'eau en complément de l'eau déjà présente dans la composition épaississante.

Le pH de la formulation aqueuse acide est inférieur à 2.

La formulation aqueuse acide ainsi réalisée s'avère trouble.

### Essai n° 5

Cet essai illustre l'invention.

### Composition épaississante

L'essai met en oeuvre une composition épaississante constituée de, exprimé en pourcentage en poids des constituants par rapport au poids total de la composition :
- 17,65 % d'un épaississant polyuréthane constitué, exprimé en pourcentage en poids des différents monomères par rapport au poids total du polymère, de :
   - 69,4 % de polyéthylène glycol de poids moléculaire égal à 5 500 g/mol,
   - 5,3 % d'isophorone di-isocyanate,
   - 25,3 % d'un alcool linéaire ayant 12 atomes de carbone,
- 15,90 % en poids d'eau,
- 21,99 % en poids d'un tensioactif non ionique éthoxylé qui est un mélange d'alcools gras ayant de 12 à 18 atomes de carbone sur la chaîne grasse, et comportant 7 motifs d'oxyde d'éthylène,
- 4,44 % en poids d'un tensioactif non ionique éthoxylé qui est un mélange d'alcools gras ayant de 12 à 18 atomes de carbone sur la chaîne grasse, et comportant 3 motifs d'oxyde d'éthylène,
- 40,02 % en poids d'un tensioactif non ionique éthoxylé qui est un mélange d'alcools gras ayant de 8 atomes de carbone sur la chaîne grasse, et comportant 35 motifs d'oxyde d'éthylène,

La composition épaississante ainsi réalisée est limpide.

### Formulation aqueuse acide

Cette composition épaississante est mise en oeuvre pour épaissir une formulation aqueuse acide, qui contient, exprimé en pourcentage en poids des constituants par rapport au poids total de la formulation :
- 3 % d'acide citrique,
- 9,1 % de la composition épaississante précitée,
- 87,9 % d'eau en complément de l'eau déjà présente dans la composition épaississante.

Le pH de la formulation aqueuse acide est inférieur à 2.

La formulation aqueuse acide ainsi réalisée s'avère limpide.

### Essai n° 6

Cet essai illustre l'invention.

### Composition épaississante

L'essai met en oeuvre une composition épaississante constituée de, exprimé en pourcentage en poids des constituants par rapport au poids total de la composition :
- 17,65 % d'un épaississant polyuréthane constitué, exprimé en pourcentage en poids des différents monomères par rapport au poids total du polymère, de :
   - 69,4 % de polyéthylène glycol de poids moléculaire égal à 8 000 g/mol,
   - 5,3 % d'isophorone di-isocyanate,
   - 25,3 % d'un alcool linéaire ayant 12 atomes de carbone,
- 15,90 % en poids d'eau,
- 21,99 % en poids d'un tensioactif non ionique éthoxylé qui est un mélange d'alcools gras ayant de 12 à 18 atomes de carbone sur la chaîne grasse, et comportant 7 motifs d'oxyde d'éthylène,
- 4,44 % en poids d'un tensioactif non ionique éthoxylé qui est un mélange d'alcools gras ayant de 12 à 18 atomes de carbone sur la chaîne grasse, et comportant 3 motifs d'oxyde d'éthylène,
- 40,02 % en poids d'un tensioactif non ionique éthoxylé qui est un mélange d'alcools gras ayant de 8 atomes de carbone sur la chaîne grasse, et comportant 35 motifs d'oxyde d'éthylène,

La composition épaississante ainsi réalisée est limpide.

### Formulation aqueuse acide

Cette composition épaississante est mise en oeuvre pour épaissir une formulation aqueuse acide, qui contient, exprimé en pourcentage en poids des constituants par rapport au poids total de la formulation :
- 3 % d'acide citrique,
- 9,1 % de la composition épaississante précitée,
- 87,9 % d'eau en complément de l'eau déjà présente dans la composition épaississante.

Le pH de la formulation aqueuse acide est inférieur à 2.

La formulation aqueuse acide ainsi réalisée s'avère limpide.

### Essai n° 7

Cet essai illustre l'invention.

### Composition épaississante

L'essai met en oeuvre une composition épaississante constituée de, exprimé en pourcentage en poids des constituants par rapport au poids total de la composition :
- 17,65 % d'un épaississant polyuréthane constitué, exprimé en pourcentage en poids des différents monomères par rapport au poids total du polymère, de :
   - 69,4% de polyéthylène glycol de poids moléculaire égal à 10 000 g/mol,
   - 5,3 % d'isophorone di-isocyanate,
   - 25,3 % d'un alcool linéaire ayant 12 atomes de carbone,
- 15,90 % en poids d'eau,
- 21,99 % en poids d'un tensioactif non ionique éthoxylé qui est un mélange d'alcools gras ayant de 12 à 18 atomes de carbone sur la chaîne grasse, et comportant 7 motifs d'oxyde d'éthylène,
- 4,44 % en poids d'un tensioactif non ionique éthoxylé qui est un mélange d'alcools gras ayant de 12 à 18 atomes de carbone sur la chaîne grasse, et comportant 3 motifs d'oxyde d'éthylène,
- 40,02 % en poids d'un tensioactif non ionique éthoxylé qui est un mélange d'alcools gras ayant de 8 atomes de carbone sur la chaîne grasse, et comportant 35 motifs d'oxyde d'éthylène.

La composition épaississante ainsi réalisée est limpide.

### Formulation aqueuse acide

Cette composition épaississante est mise en oeuvre pour épaissir une formulation aqueuse acide, qui contient, exprimé en pourcentage en poids des constituants par rapport au poids total de la formulation :
- 3 % d'acide citrique,
- 9,1 % de la composition épaississante précitée,
- 87,9 % d'eau en complément de l'eau déjà présente dans la composition épaississante.

Le pH de la formulation aqueuse acide est inférieur à 2.

La formulation aqueuse acide ainsi réalisée s'avère limpide.

### Essai n° 8

Cet essai illustre l'invention.

### Composition épaississante

L'essai met en oeuvre une composition épaississante constituée de, exprimé en pourcentage en poids des constituants par rapport au poids total de la composition :
- 17,65 % d'un épaississant polyuréthane constitué, exprimé en pourcentage en poids des différents monomères par rapport au poids total du polymère, de :
   - 88,5 % de polyéthylène glycol de poids moléculaire égal à 10 000 g/mol,
   - 5,4 % d'isophorone di-isocyanate,
   - 6,1 % d'un mélange d'alcools linéaires ayant 12 et 13 atomes de carbone,
- 15,90 % en poids d'eau,
- 21,99 % en poids d'un tensioactif non ionique éthoxylé qui est un mélange d'alcools gras ayant de 12 à 18 atomes de carbone sur la chaîne grasse, et comportant 7 motifs d'oxyde d'éthylène,
- 4,44 % en poids d'un tensioactif non ionique éthoxylé qui est un mélange d'alcools gras ayant de 12 à 18 atomes de carbone sur la chaîne grasse, et comportant 3 motifs d'oxyde d'éthylène,
- 40,02 % en poids d'un tensioactif non ionique éthoxylé qui est un mélange d'alcools gras ayant de 8 atomes de carbone sur la chaîne grasse, et comportant 35 motifs d'oxyde d'éthylène.

La composition épaississante ainsi réalisée est limpide.

### Formulation aqueuse acide

Cette composition épaississante est mise en oeuvre pour épaissir une formulation aqueuse acide, qui contient, exprimé en pourcentage en poids des constituants par rapport au poids total de la formulation :
- 3 % d'acide citrique,
- 9,1 % de la composition épaississante précitée,
- 87,9 % d'eau en complément de l'eau déjà présente dans la composition épaississante.

Le pH de la formulation aqueuse acide est inférieur à 2.

La formulation aqueuse acide ainsi réalisée s'avère limpide.

### Essai n° 9

Cet essai illustre l'invention.

### Composition épaississante

L'essai met en oeuvre une composition épaississante constituée de, exprimé en pourcentage en poids des constituants par rapport au poids total de la composition :
- 17,65 % d'un épaississant polyuréthane constitué, exprimé en pourcentage en poids des différents monomères par rapport au poids total du polymère, de :
   - 87,2 % de polyéthylène glycol de poids moléculaire égal à 10 000 g/mol,
   - 6,6 % d'isophorone di-isocyanate,
   - 6,2 % d'un mélange d'alcools linéaires ayant 12, 13, 14 et 15 atomes de carbone,
- 15,90 % en poids d'eau,
- 21,99 % en poids d'un tensioactif non ionique éthoxylé qui est un mélange d'alcools gras ayant de 12 à 18 atomes de carbone sur la chaîne grasse, et comportant 7 motifs d'oxyde d'éthylène,
- 4,44 % en poids d'un tensioactif non ionique éthoxylé qui est un mélange d'alcools gras ayant de 12 à 18 atomes de carbone sur la chaîne grasse, et comportant 3 motifs d'oxyde d'éthylène,
- 40,02 % en poids d'un tensioactif non ionique éthoxylé qui est un mélange d'alcools gras ayant de 8 atomes de carbone sur la chaîne grasse, et comportant 35 motifs d'oxyde d'éthylène.

La composition épaississante ainsi réalisée est limpide.

### Formulation aqueuse acide

Cette composition épaississante est mise en oeuvre pour épaissir une formulation aqueuse acide, qui contient, exprimé en pourcentage en poids des constituants par rapport au poids total de la formulation :
- 3 % d'acide citrique,
- 9,1 % de la composition épaississante précitée,
- 87,9 % d'eau en complément de l'eau déjà présente dans la composition épaississante.

Le pH de la formulation aqueuse acide est inférieur à 2.

La formulation aqueuse acide ainsi réalisée s'avère limpide.

### Essai n° 10

Cet essai illustre l'invention.

### Composition épaississante

L'essai met en oeuvre une composition épaississante constituée de, exprimé en pourcentage en poids des constituants par rapport au poids total de la composition :
- 17,65 % d'un épaississant polyuréthane constitué, exprimé en pourcentage en poids des différents monomères par rapport au poids total du polymère, de :
   - 88,2 % de polyéthylène glycol de poids moléculaire égal à 10 000 g/mol,
   - 6,0 % d'isophorone di-isocyanate,
   - 5,8 % d'un alcool linéaire ayant 14 atomes de carbone,
- 15,90 % en poids d'eau,
- 21,99 % en poids d'un tensioactif non ionique éthoxylé qui est un mélange d'alcools gras ayant de 12 à 18 atomes de carbone sur la chaîne grasse, et comportant 7 motifs d'oxyde d'éthylène,
- 4,44 % en poids d'un tensioactif non ionique éthoxylé qui est un mélange d'alcools gras ayant de 12 à 18 atomes de carbone sur la chaîne grasse, et comportant 3 motifs d'oxyde d'éthylène,
- 40,02 % en poids d'un tensioactif non ionique éthoxylé qui est un mélange d'alcools gras ayant de 8 atomes de carbone sur la chaîne grasse, et comportant 35 motifs d'oxyde d'éthylène.

La composition épaississante ainsi réalisée est limpide.

### Formulation aqueuse acide

Cette composition épaississante est mise en oeuvre pour épaissir une formulation aqueuse acide, qui contient, exprimé en pourcentage en poids des constituants par rapport au poids total de la formulation :
- 3 % d'acide citrique,
- 5,7 % de la composition épaississante précitée,
- 91,3 % d'eau en complément de l'eau déjà présente dans la composition épaississante.

Le pH de la formulation aqueuse acide est inférieur à 2.

La formulation aqueuse acide ainsi réalisée s'avère limpide.

### Essai n° 11

Cet essai illustre l'invention.

### Composition épaississante

L'essai met en oeuvre une composition épaississante constituée de, exprimé en pourcentage en poids des constituants par rapport au poids total de la composition :
- 17,65 % d'un épaississant polyuréthane constitué, exprimé en pourcentage en poids des différents monomères par rapport au poids total du polymère, de :
   - 88,2 % de polyéthylène glycol de poids moléculaire égal à 10 000 g/mol,
   - 6,0 % d'isophorone di-isocyanate,
   - 5,8 % d'un alcool linéaire ayant 14 atomes de carbone,
- 15,90 % en poids d'eau,
- 21,99 % en poids d'un tensioactif non ionique éthoxylé qui est un mélange d'alcools gras ayant de 12 à 18 atomes de carbone sur la chaîne grasse, et comportant 7 motifs d'oxyde d'éthylène,
- 4,44 % en poids d'un tensioactif non ionique éthoxylé qui est un mélange d'alcools gras ayant de 12 à 18 atomes de carbone sur la chaîne grasse, et comportant 3 motifs d'oxyde d'éthylène,
- 40,02 % en poids d'un tensioactif non ionique éthoxylé qui est un mélange d'alcools gras ayant de 8 atomes de carbone sur la chaîne grasse, et comportant 35 motifs d'oxyde d'éthylène.

La composition épaississante ainsi réalisée est limpide.

### Formulation aqueuse acide

Cette composition épaississante est mise en oeuvre pour épaissir une formulation aqueuse acide, qui contient, exprimé en pourcentage en poids des constituants par rapport au poids total de la formulation :
- 3 % d'acide citrique,
- 4,35 % de la composition épaississante précitée,
- 92,65 % d'eau en complément de l'eau déjà présente dans la composition épaississante.

Le pH de la formulation aqueuse acide est inférieur à 2.

La formulation aqueuse acide ainsi réalisée s'avère limpide.

### Essai n° 12

Cet essai illustre l'invention.

### Composition épaississante

L'essai met en oeuvre une composition épaississante constituée de, exprimé en pourcentage en poids des constituants par rapport au poids total de la composition :
- 17,65 % d'un épaississant polyuréthane constitué, exprimé en pourcentage en poids des différents monomères par rapport au poids total du polymère, de :
   - 88,2 % de polyéthylène glycol de poids moléculaire égal à 10 000 g/mol,
   - 6,0 % d'isophorone di-isocyanate,
   - 5,8 % d'un alcool linéaire ayant 14 atomes de carbone,
- 15,90 % en poids d'eau,
- 21,99 % en poids d'un tensioactif non ionique éthoxylé qui est un mélange d'alcools gras ayant de 12 à 18 atomes de carbone sur la chaîne grasse, et comportant 7 motifs d'oxyde d'éthylène,
- 4,44 % en poids d'un tensioactif non ionique éthoxylé qui est un mélange d'alcools gras ayant de 12 à 18 atomes de carbone sur la chaîne grasse, et comportant 3 motifs d'oxyde d'éthylène,
- 40,02 % en poids d'un tensioactif non ionique éthoxylé qui est un mélange d'alcools gras ayant de 8 atomes de carbone sur la chaîne grasse, et comportant 35 motifs d'oxyde d'éthylène.

La composition épaississante ainsi réalisée est limpide.

### Formulation aqueuse acide

Cette composition épaississante est mise en oeuvre pour épaissir une formulation aqueuse acide, qui contient, exprimé en pourcentage en poids des constituants par rapport au poids total de la formulation :
- 3 % d'acide citrique,
- 4,4 % de la composition épaississante précitée,
- 92,6 % d'eau en complément de l'eau déjà présente dans la composition épaississante.

Le pH de la formulation aqueuse acide est inférieur à 2.

La formulation aqueuse acide ainsi réalisée s'avère limpide.

### Essai n° 13

Cet essai illustre l'invention.

### Composition épaississante

L'essai met en oeuvre une composition épaississante constituée de, exprimé en pourcentage en poids des constituants par rapport au poids total de la composition :
- 17,65 % d'un épaississant polyuréthane constitué, exprimé en pourcentage en poids des différents monomères par rapport au poids total du polymère, de :
   - 88,2 % de polyéthylène glycol de poids moléculaire égal à 10 000 g/mol,
   - 6,0 % d'isophorone di-isocyanate,
   - 5,8 % d'un alcool linéaire ayant 14 atomes de carbone,
- 15,90 % en poids d'eau,
- 21,99 % en poids d'un tensioactif non ionique éthoxylé qui est un mélange d'alcools gras ayant de 12 à 18 atomes de carbone sur la chaîne grasse, et comportant 7 motifs d'oxyde d'éthylène,
- 4,44 % en poids d'un tensioactif non ionique éthoxylé qui est un mélange d'alcools gras ayant de 12 à 18 atomes de carbone sur la chaîne grasse, et comportant 3 motifs d'oxyde d'éthylène,
- 40,02 % en poids d'un tensioactif non ionique éthoxylé qui est un mélange d'alcools gras ayant de 8 atomes de carbone sur la chaîne grasse, et comportant 35 motifs d'oxyde d'éthylène.

La composition épaississante ainsi réalisée est limpide.

### Formulation aqueuse acide

Cette composition épaississante est mise en oeuvre pour épaissir une formulation aqueuse acide, qui contient, exprimé en pourcentage en poids des constituants par rapport au poids total de la formulation :
- 3 % d'acide citrique,
- 4,45 % de la composition épaississante précitée,
- 92,55 % d'eau en complément de l'eau déjà présente dans la composition épaississante.

Le pH de la formulation aqueuse acide est inférieur à 2.

La formulation aqueuse acide ainsi réalisée s'avère limpide.

### Essai n° 14

Cet essai illustre l'invention.

### Composition épaississante

L'essai met en oeuvre une composition épaississante constituée de, exprimé en pourcentage en poids des constituants par rapport au poids total de la composition :
- 17,65 % d'un épaississant polyuréthane constitué, exprimé en pourcentage en poids des différents monomères par rapport au poids total du polymère, de :
   - 88,2 % de polyéthylène glycol de poids moléculaire égal à 10 000 g/mol,
   - 6,0 % d'isophorone di-isocyanate,
   - 5,8 % d'un alcool linéaire ayant 14 atomes de carbone,
- 15,90 % en poids d'eau,
- 21,99 % en poids d'un tensioactif non ionique éthoxylé qui est un mélange d'alcools gras ayant de 12 à 18 atomes de carbone sur la chaîne grasse, et comportant 7 motifs d'oxyde d'éthylène,
- 4,44 % en poids d'un tensioactif non ionique éthoxylé qui est un mélange d'alcools gras ayant de 12 à 18 atomes de carbone sur la chaîne grasse, et comportant 3 motifs d'oxyde d'éthylène,
- 40,02 % en poids d'un tensioactif non ionique éthoxylé qui est un mélange d'alcools gras ayant de 8 atomes de carbone sur la chaîne grasse, et comportant 35 motifs d'oxyde d'éthylène.

La composition épaississante ainsi réalisée est limpide.

### Formulation aqueuse acide

Cette composition épaississante est mise en oeuvre pour épaissir une formulation aqueuse acide, qui contient, exprimé en pourcentage en poids des constituants par rapport au poids total de la formulation :
- 3 % d'acide citrique,
- 4,5 % de la composition épaississante précitée,
- 92,5 % d'eau en complément de l'eau déjà présente dans la composition épaississante.

Le pH de la formulation aqueuse acide est inférieur à 2.

La formulation aqueuse acide ainsi réalisée s'avère limpide.

### Essai n° 15

Cet essai illustre l'invention.

### Composition épaississante

L'essai met en oeuvre une composition épaississante constituée de, exprimé en pourcentage en poids des constituants par rapport au poids total de la composition :
- 17,65 % d'un épaississant polyuréthane constitué, exprimé en pourcentage en poids des différents monomères par rapport au poids total du polymère, de :
   - 86,6 % de polyéthylène glycol de poids moléculaire égal à 10 000 g/mol,
   - 6,7 % d'isophorone di-isocyanate,
   - 6,7 % d'un alcool linéaire ayant 14 atomes de carbone,
- 15,90 % en poids d'eau,
- 21,99 % en poids d'un tensioactif non ionique éthoxylé qui est un mélange d'alcools gras ayant de 12 à 18 atomes de carbone sur la chaîne grasse, et comportant 7 motifs d'oxyde d'éthylène,
- 4,44 % en poids d'un tensioactif non ionique éthoxylé qui est un mélange d'alcools gras ayant de 12 à 18 atomes de carbone sur la chaîne grasse, et comportant 3 motifs d'oxyde d'éthylène,
- 40,02 % en poids d'un tensioactif non ionique éthoxylé qui est un mélange d'alcools gras ayant de 8 atomes de carbone sur la chaîne grasse, et comportant 35 motifs d'oxyde d'éthylène.

La composition épaississante ainsi réalisée est limpide.

### Formulation aqueuse acide

Cette composition épaississante est mise en oeuvre pour épaissir une formulation aqueuse acide, qui contient, exprimé en pourcentage en poids des constituants par rapport au poids total de la formulation :
- 3 % d'acide citrique,
- 5,7 % de la composition épaississante précitée,
- 91,2 % d'eau en complément de l'eau déjà présente dans la composition épaississante.

Le pH de la formulation aqueuse acide est inférieur à 2.

La formulation aqueuse acide ainsi réalisée s'avère limpide.

### Essai n° 16

Cet essai illustre l'invention.

### Composition épaississante

L'essai met en oeuvre une composition épaississante constituée de, exprimé en pourcentage en poids des constituants par rapport au poids total de la composition :
- 17,65 % d'un épaississant polyuréthane constitué, exprimé en pourcentage en poids des différents monomères par rapport au poids total du polymère, de :
   - 86,6 % de polyéthylène glycol de poids moléculaire égal à 10 000 g/mol,
   - 6,7 % d'isophorone di-isocyanate,
   - 6,7 % d'un alcool linéaire ayant 14 atomes de carbone,
- 15,90 % en poids d'eau,
- 21,99 % en poids d'un tensioactif non ionique éthoxylé qui est un mélange d'alcools gras ayant de 12 à 18 atomes de carbone sur la chaîne grasse, et comportant 7 motifs d'oxyde d'éthylène,
- 4,44 % en poids d'un tensioactif non ionique éthoxylé qui est un mélange d'alcools gras ayant de 12 à 18 atomes de carbone sur la chaîne grasse, et comportant 3 motifs d'oxyde d'éthylène,
- 40,02 % en poids d'un tensioactif non ionique éthoxylé qui est un mélange d'alcools gras ayant de 8 atomes de carbone sur la chaîne grasse, et comportant 35 motifs d'oxyde d'éthylène.

La composition épaississante ainsi réalisée est limpide.

### Formulation aqueuse acide

Cette composition épaississante est mise en oeuvre pour épaissir une formulation aqueuse acide, qui contient, exprimé en pourcentage en poids des constituants par rapport au poids total de la formulation :
- 3 % d'acide citrique,
- 4,5 % de la composition épaississante précitée,
- 92,5% d'eau en complément de l'eau déjà présente dans la composition épaississante.

Le pH de la formulation aqueuse acide est inférieur à 2.
La formulation aqueuse acide ainsi réalisée s'avère limpide.

L'ensemble des résultats relatifs aux essais n° 1 à 16 figure dans le tableau 1.

### TABLEAU 1

**Tableau 1 : caractéristiques des compositions aqueuses épaississantes et des formulations aqueuses acides**

| essai n° | art antérieur / invention | formulation aqueuse acide (quantité des constituants en % en poids par rapport au poids total formulation aqueuse acide) | | | aspect de la composition épaississante | aspect de la formulation aqueuse acide | viscosité Brookfield™ de la formulation (mPa.s) | R= viscosité Brookfield™ / masse composition épaississante |
|---|---|---|---|---|---|---|---|---|
| | | acide citrique | composition épaississante | eau | limpide / trouble | limpide / trouble | | |
| 1 | art antérieur | 3 | 14,0 | 83,0 | trouble | trouble | 30 | 2 |
| 2 | art antérieur | 3 | 14,1 | 82,9 | trouble | trouble | 275 | 20 |
| 3 | art antérieur | 3 | 13,6 | 83,4 | trouble | trouble | 25 | 2 |
| 4 | art antérieur | 3 | 10,4 | 86,6 | trouble | trouble | 525 | 50 |
| 5 | invention | 3 | 9,1 | 87,9 | limpide | limpide | 875 | 96 |
| 6 | invention | 3 | 9,1 | 87,9 | limpide | limpide | 650 | 71 |
| 7 | invention | 3 | 9,1 | 87,9 | limpide | limpide | 1200 | 132 |
| 8 | invention | 3 | 9,1 | 91,3 | limpide | limpide | 1400 | 154 |
| 9 | invention | 3 | 9,1 | 91,3 | limpide | limpide | 1550 | 170 |
| 10 | invention | 3 | 5,7 | 91,3 | limpide | limpide | 830 | 146 |
| 11 | invention | 3 | 4,35 | 92,7 | limpide | limpide | 290 | 67 |
| 12 | invention | 3 | 4,4 | 92,6 | limpide | limpide | 360 | 82 |
| 13 | invention | 3 | 4,45 | 92,6 | limpide | limpide | 410 | 92 |
| 14 | invention | 3 | 4,5 | 92,5 | limpide | limpide | 470 | 104 |
| 15 | invention | 3 | 5,7 | 91,3 | limpide | limpide | 870 | 153 |
| 16 | invention | 3 | 4,5 | 92,5 | limpide | limpide | 400 | 89 |

La lecture du tableau 1 démontre que les compositions aqueuses épaississantes de l'art antérieur ne donnent pas satisfaction à l'homme du métier : elles sont toutes troubles et il en est de même pour les formulations aqueuses acides qui les contiennent ; d'autre part, leur effet épaississant est faible, comme en témoignent les valeurs du rapport R toutes inférieures à 50.

En revanche, les compositions aqueuses épaississantes selon l'invention, qui reposent notamment sur un choix particulier de tensioactifs et de composés polyuréthannes, possèdent un caractère limpide, de même que les formulations aqueuses acides qui les contiennent. De plus, l'effet épaississant obtenu est bien meilleur que celui mesuré pour l'art antérieur, puisque les valeurs de R sont toutes supérieures à 50 dans le cas de l'invention.

### Exemple 2

Cet exemple illustre l'utilisation d'une composition aqueuse épaississante selon l'invention, en vue d'épaissir une formulation aqueuse acide contenant de l'acide citrique ou de l'eau oxygénée.

Cet exemple illustre également les compositions aqueuses épaississantes selon l'invention, ainsi que les formulations aqueuses acide selon l'invention.

Pour les essais n° 17 et 18, on commence par évaluer visuellement le caractère limpide ou trouble de chaque composition épaississante, et de chaque formulation aqueuse acide.

Enfin, on mesure la viscosité Brookfield™ de chaque formulation aqueuse acide, à 25°C et à 10 tours par minute avec le module n° 3 (selon la méthode bien connue de l'homme du métier) à l'instant t=0. on réalise la même mesure à l'instant t=28 jours.

### Essai n° 17

Cet essai illustre l'invention.

### Composition épaississante

L'essai met en oeuvre une composition épaississante constituée de, exprimé en pourcentage en poids des constituants par rapport au poids total de la composition :
- 17,65 % d'un épaississant polyuréthane constitué, exprimé en pourcentage en poids des différents monomères par rapport au poids total du polymère, de :
   - 86,6 % de polyéthylène glycol de poids moléculaire égal à 10 000 g/mol,
   - 6,7 % d'isophorone di-isocyanate,
   - 6,7 % d'un alcool linéaire ayant 14 atomes de carbone,
- 15,90 % en poids d'eau,
- 21,99 % en poids d'un tensioactif non ionique éthoxylé qui est un mélange d'alcools gras ayant de 12 à 18 atomes de carbone sur la chaîne grasse, et comportant 7 motifs d'oxyde d'éthylène,
- 4,44 % en poids d'un tensioactif non ionique éthoxylé qui est un mélange d'alcools gras ayant de 12 à 18 atomes de carbone sur la chaîne grasse, et comportant 3 motifs d'oxyde d'éthylène,
- 40,02 % en poids d'un tensioactif non ionique éthoxylé qui est un mélange d'alcools gras ayant de 8 atomes de carbone sur la chaîne grasse, et comportant 35 motifs d'oxyde d'éthylène.

La composition épaississante ainsi réalisée est limpide.

### Formulation aqueuse acide

Cette composition épaississante est mise en oeuvre pour épaissir une formulation aqueuse acide, qui contient, exprimé en pourcentage en poids des constituants par rapport au poids total de la formulation :
- 10 % d'acide citrique,
- 10,4 % de la composition épaississante précitée,
- 79,6 % d'eau en complément de l'eau déjà présente dans la composition épaississante.

Le pH de la formulation aqueuse acide est inférieur à 2.

La formulation aqueuse acide ainsi réalisée s'avère limpide.

La viscosité Brookfield™ de la formulation aqueuse acide, à 25°C et à 10 tours par minute avec le module n° 3 (selon la méthode bien connue de l'homme du métier) est égale à :
- 1275 mPa.s à l'instant t=0
- 1200 mPa.s à l'instant t=28 jours

Cet essai démontre donc que la composition aqueuse selon l'invention permet d'épaissir efficacement la formulation aqueuse acide et ce, tout en maintenant son caractère limpide.

D'autre part, cet essai démontre aussi que l'effet d'épaississement peut être maintenu au cours du temps grâce à la composition aqueuse épaississante selon l'invention.

### Essai n° 18

Cet essai illustre l'invention.

### Composition épaississante

L'essai met en oeuvre une composition épaississante constituée de, exprimé en pourcentage en poids des constituants par rapport au poids total de la composition :
- 17,65 % d'un épaississant polyuréthane constitué, exprimé en pourcentage en poids des différents monomères par rapport au poids total du polymère, de :
   - 86,6 % de polyéthylène glycol de poids moléculaire égal à 10 000 g/mol,
   - 6,7 % d'isophorone di-isocyanate,
   - 6,7 % d'un alcool linéaire ayant 14 atomes de carbone,
- 15,90 % en poids d'eau,
- 21,99 % en poids d'un tensioactif non ionique éthoxylé qui est un mélange d'alcools gras ayant de 12 à 18 atomes de carbone sur la chaîne grasse, et comportant 7 motifs d'oxyde d'éthylène,
- 4,44 % en poids d'un tensioactif non ionique éthoxylé qui est un mélange d'alcools gras ayant de 12 à 18 atomes de carbone sur la chaîne grasse, et comportant 3 motifs d'oxyde d'éthylène,
- 40,02 % en poids d'un tensioactif non ionique éthoxylé qui est un mélange d'alcools gras ayant de 8 atomes de carbone sur la chaîne grasse, et comportant 35 motifs d'oxyde d'éthylène.

La composition épaississante ainsi réalisée est limpide.

### Formulation aqueuse acide

Cette composition épaississante est mise en oeuvre pour épaissir une formulation aqueuse acide, qui contient, exprimé en pourcentage en poids des constituants par rapport au poids total de la formulation :
- 21,1 % d'eau oxygénée,
- 11,0 % de la composition épaississante précitée,
- 0,5 % d'acide 1-hydroxy éthylène 1,1 di-phosphonique utilisé comme stabilisant de l'eau oxygénée
- 67,6 % d'eau en complément de l'eau déjà présente dans la composition épaississante.

Le pH de la formulation aqueuse acide est inférieur à 3.

La formulation aqueuse acide ainsi réalisée s'avère limpide.

La viscosité Brookfield™ de la formulation aqueuse acide, à 25°C et à 10 tours par minute avec le module n° 3 (selon la méthode bien connue de l'homme du métier) est égale à :
- 2100 mPa.s à l'instant t=0
- 1800 mPa.s à l'instant t=28 jours

Cet essai démontre donc que la composition aqueuse selon l'invention permet d'épaissir efficacement la formulation aqueuse acide et ce, tout en maintenant son caractère limpide.

D'autre part, cet essai démontre aussi que l'effet d'épaississement peut être maintenu au cours du temps grâce à la composition aqueuse épaississante selon l'invention.

### Exemple 3

Les essais n° 19 à 23 qui illustrent l'invention mettent en oeuvre une composition épaississante constituée de, exprimé en pourcentage en poids des constituants par rapport au poids total de la composition :
- 17,65 % d'un épaississant polyuréthane constitué, exprimé en pourcentage en poids des différents monomères par rapport au poids total du polymère, de :
   - 81,5 % de polyéthylène glycol de poids moléculaire égal à 6 000 g/mol,
   - 6,7 % d'isophorone di-isocyanate,
   - 6,7 % d'un alcool linéaire ayant 12 atomes de carbone,
- 15,90 % en poids d'eau,
- 21,99 % en poids d'un tensioactif non ionique éthoxylé qui est un mélange d'alcools gras ayant de 12 à 18 atomes de carbone sur la chaîne grasse, et comportant 7 motifs d'oxyde d'éthylène,
- 4,44 % en poids d'un tensioactif non ionique éthoxylé qui est un mélange d'alcools gras ayant de 12 à 18 atomes de carbone sur la chaîne grasse, et comportant 3 motifs d'oxyde d'éthylène,
- 40,02 % en poids d'un tensioactif non ionique éthoxylé qui est un mélange d'alcools gras ayant de 8 atomes de carbone sur la chaîne grasse, et comportant 35 motifs d'oxyde d'éthylène.

La composition épaississante ainsi réalisée est limpide.

Cette composition épaississante est utilisée pour épaissir différentes formulations aqueuses acides, dont la nature est donnée dans le tableau 2.

Ce tableau indique également la valeur de la viscosité Brookfield™ de chaque formulation aqueuse acide, à 25°C et à 10 tours par minute avec le module n° 3 (selon la méthode bien connue de l'homme du métier), ainsi que son caractère limpide ou trouble.

**Tableau 2 : formulations aqueuses acides avec :**

| **essai n°** | 19 | 20 | 21 | 22 | 23 |
|---|---|---|---|---|---|
| **composition épaississante** | 9,1 | 7,65 | 9,1 | 6,5 | 7,88 |
| **acide** | 3 (citrique) | 3 (borique) | 3 (formique) | 3 (lactique) | 3 (HEDP) |
| **eau** | 87,9 | 89,35 | 87,9 | 90,5 | 89,12 |
| **viscosité Brookfield 10 t/min (mPa.s)** | 740 | 310 | 520 | 125 | 320 |
| **aspect** | limpide | limpide | limpide | limpide | limpide |

- quantité de chaque constituant exprimée en pourcentage en poids par rapport au poids total de la formulation
- nature de l'acide mis en oeuvre (entre parenthèses)
- HEDP : acide 1-hydroxy éthylène 1,1 di-phosphonique

Ces essais démontrent donc que les compositions aqueuses selon l'invention permettent d'épaissir efficacement les formulations aqueuses acides selon l'invention et ce, tout en maintenant leur caractère limpide.

### Exemple 4

Les essais n° 24 à 27 qui illustrent l'invention mettent en oeuvre une composition épaississante constituée de, exprimé en pourcentage en poids des constituants par rapport au poids total de la composition :
- 30,0 % d'un épaississant polyuréthane constitué, exprimé en pourcentage en poids des différents monomères par rapport au poids total du polymère, de :
   - 81,5 % de polyéthylène glycol de poids moléculaire égal à 6 000 g/mol,
   - 6,7 % d'isophorone di-isocyanate,
   - 6,7 % d'un alcool linéaire ayant 12 atomes de carbone,
- 20,0 % en poids d'eau,
- 20,0 % en poids d'un tensioactif non ionique éthoxylé qui est un mélange d'alcools gras ayant de 9 atomes de carbone sur la chaîne grasse, et comportant 115 motifs d'oxyde d'éthylène.
- 30,0 % en poids d'un tensioactif non ionique éthoxylé qui est un mélange d'alcools gras ayant de 8 atomes de carbone sur la chaîne grasse, et comportant 35 motifs d'oxyde d'éthylène.

La composition épaississante ainsi réalisée est limpide.

Cette composition épaississante est utilisée pour épaissir différentes formulations aqueuses acides, dont la nature est donnée dans le tableau 3.

Ce tableau indique également la valeur de la viscosité Brookfield™ de chaque formulation aqueuse acide, à 25°C et à 10 tours par minute avec le module n° 3 (selon la méthode bien connue de l'homme du métier), ainsi que son caractère limpide ou trouble.

**Tableau 3 : formulations aqueuses acides avec :**

| **essai n°** | 24 | 25 | 26 | 27 |
|---|---|---|---|---|
| **composition épaississante** | 9,1 | 6,47 | 8 | 6 |
| **acide** | 3 (phosphorique) | 3 (citrique) | 3 (nitrique) | 3 (acétique) |
| **eau** | 87,9 | 90,53 | 89 | 91 |
| **viscosité Brookfield 10 t/min (mPa.s)** | 2500 | 240 | 680 | 160 |
| **aspect** | limpide | limpide | limpide | limpide |

- quantité de chaque constituant exprimée en pourcentage en poids par rapport au poids total de la formulation
- nature de l'acide mis en oeuvre (entre parenthèses)

Ces essais démontrent donc que les compositions aqueuses selon l'invention permettent d'épaissir efficacement les formulations aqueuses acides selon l'invention et ce, tout en maintenant leur caractère limpide.

### Exemple 5

Les essais n° 28 à 29 qui illustrent l'invention mettent en oeuvre une composition épaississante constituée de, exprimé en pourcentage en poids des constituants par rapport au poids total de la composition :
- 25,0 % d'un épaississant polyuréthane constitué, exprimé en pourcentage en poids des différents monomères par rapport au poids total du polymère, de :
   - 81,5 % de polyéthylène glycol de poids moléculaire égal à 6 000 g/mol,
   - 6,7 % d'isophorone di-isocyanate,
   - 6,7 % d'un alcool linéaire ayant 12 atomes de carbone,
- 31,0 % en poids d'eau,
- 25,0 % en poids d'un tensioactif non ionique éthoxylé qui est un mélange d'alcools gras ayant de 8 atomes de carbone sur la chaîne grasse, et comportant 35 motifs d'oxyde d'éthylène,
- 15,0 % en poids d'un tensioactif non ionique éthoxylé qui est un mélange d'alcools gras ayant de 14 à 15 atomes de carbone sur la chaîne grasse, et comportant 7 motifs d'oxyde d'éthylène,
- 4,0 % en poids d'un tensioactif non ionique éthoxylé qui est un mélange d'alcools gras ayant de 12 à 18 atomes de carbone sur la chaîne grasse, et comportant 3 motifs d'oxyde d'éthylène.

La composition épaississante ainsi réalisée est limpide.

Cette composition épaississante est utilisée pour épaissir différentes formulations aqueuses acides, dont la nature est donnée dans le tableau 4.

Ce tableau indique également la valeur de la viscosité Brookfield™ de chaque formulation aqueuse acide, à 25°C et à 10 tours par minute avec le module n° 3 (selon la méthode bien connue de l'homme du métier), ainsi que son caractère limpide ou trouble.

**Tableau 4 : formulations aqueuses acides avec :**

| **essai n°** | 28 | 29 |
|---|---|---|
| **composition épaississante** | 11,98 | 9,4 |
| **acide** | 3 (PBTC) | 3 (chlorhydrique) |
| **eau** | 85,02 | 87,6 |
| **viscosité Brookfield 10 t/min (mPa.s)** | 1540 | 400 |
| **aspect** | limpide | limpide |

- quantité de chaque constituant exprimée en pourcentage en poids par rapport au poids total de la formulation
- nature de l'acide mis en oeuvre (entre parenthèses)
- PBTC : acide 2-phosphonobutane-1,2,4-tricarboxylique

Ces essais démontrent donc que les compositions aqueuses selon l'invention permettent d'épaissir efficacement les formulations aqueuses acides selon l'invention et ce, tout en maintenant leur caractère limpide.

### Exemple 6

Les essais n° 30 à 33 qui illustrent l'invention mettent en oeuvre une composition épaississante constituée de, exprimé en pourcentage en poids des constituants par rapport au poids total de la composition :
- 10,0 % d'un épaississant polyuréthane constitué, exprimé en pourcentage en poids des différents monomères par rapport au poids total du polymère, de :
   - 93,7 % de polyéthylène glycol de poids moléculaire égal à 20 000 g/mol,
   - 3,43 % d'isophorone di-isocyanate,
   - 2,87 % d'un alcool linéaire ayant 12 atomes de carbone,
- 20,0 % en poids d'eau,
- 40,0 % en poids d'un tensioactif non ionique éthoxylé qui est un mélange d'alcools gras ayant de 8 à 10 atomes de carbone sur la chaîne grasse, et comportant 100 motifs d'oxyde d'éthylène et 5 motifs d'oxyde de propylène,
- 30,0 % en poids d'un tensioactif non ionique éthoxylé qui est un mélange d'alcools gras ayant de 12 à 15 atomes de carbone sur la chaîne grasse, et comportant 7 motifs d'oxyde d'éthylène.

La composition épaississante ainsi réalisée est limpide.

Cette composition épaississante est utilisée pour épaissir différentes formulations aqueuses acides, dont la nature est donnée dans le tableau 5.

Ce tableau indique également la valeur de la viscosité Brookfield™ de chaque formulation aqueuse acide, à 25°C et à 10 tours par minute avec le module n° 3 (selon la méthode bien connue de l'homme du métier), ainsi que son caractère limpide ou trouble.

**Tableau 5 : formulations aqueuses acides avec :**

| **essai n°** | 30 | 31 | 32 | 33 |
|---|---|---|---|---|
| **composition épaississante** | 16 | 9,1 | 16,15 | 9,3 |
| **acide** | 3 (citrique) | 3 (oxalique) | 3 (succinique) | (7,4) eau oxygénée* |
| **eau** | 81 | 87,9 | 80,85 | 83,16 |
| **viscosité Brookfield 10 t/min (mPa.s)** | 270 | 312 | 204 | 2060 |
| **aspect** | limpide | limpide | limpide | limpide |

- quantité de chaque constituant exprimée en pourcentage en poids par rapport au poids total de la formulation
- nature de l'acide mis en oeuvre (entre parenthèses)
- * l'eau oxygénée est stabilisée avec 0,14 % en poids (par rapport au poids total de la formulation) d'acide 1-hydroxy éthylène 1,1 di-phosphonique

Ces essais démontrent donc que les compositions aqueuses selon l'invention permettent d'épaissir efficacement les formulations aqueuses acides selon l'invention et ce, tout en maintenant leur caractère limpide.

### Exemple 7

Les essais n° 34 à 36 qui illustrent l'invention mettent en oeuvre une composition épaississante constituée de, exprimé en pourcentage en poids des constituants par rapport au poids total de la composition :
- 17,65 % d'un épaississant polyuréthane constitué, exprimé en pourcentage en poids des différents monomères par rapport au poids total du polymère, de :
   - 93,7 % de polyéthylène glycol de poids moléculaire égal à 20 000 g/mol,
   - 3,43 % d'isophorone di-isocyanate,
   - 2,87 % d'un alcool linéaire ayant 12 atomes de carbone,
- 35,91 % en poids d'eau,
- 20,0 % en poids d'un tensioactif non ionique éthoxylé qui est un mélange d'alcools gras ayant 8 atomes de carbone sur la chaîne grasse, et comportant 35 motifs d'oxyde d'éthylène,
- 22,0 % en poids d'un tensioactif non ionique éthoxylé qui est un mélange d'alcools gras ayant de 12 à 18 atomes de carbone sur la chaîne grasse, et comportant 7 motifs d'oxyde d'éthylène,
- 4,44 % en poids d'un tensioactif non ionique éthoxylé qui est un mélange d'alcools gras ayant de 12 à 18 atomes de carbone sur la chaîne grasse, et comportant 3 motifs d'oxyde d'éthylène.

La composition épaississante ainsi réalisée est limpide.

Cette composition épaississante est utilisée pour épaissir différentes formulations aqueuses acides, dont la nature est donnée dans le tableau 6.

Ce tableau indique également la valeur de la viscosité Brookfield™ de chaque formulation aqueuse acide, à 25°C et à 10 tours par minute avec le module n° 3 (selon la méthode bien connue de l'homme du métier), ainsi que son caractère limpide ou trouble.

**Tableau 6 : formulations aqueuses acides avec :**

| **essai n°** | 34 | 35 | 36 |
|---|---|---|---|
| **composition épaississante** | 15,35 | 18,82 | 17 |
| **acide** | 3 (EDTMP) | 3 (itaconique) | 3 (sulfurique) |
| **eau** | 81,65 | 78,18 | 80 |
| **viscosité Brookfield 10 t/min (mPa.s)** | 80 | 40 | 60 |
| **aspect** | limpide | limpide | limpide |

- quantité de chaque constituant exprimée en pourcentage en poids par rapport au poids total de la formulation
- nature de l'acide mis en oeuvre (entre parenthèses)
- EDTMP : éthylène diamine tétraméthyl phosphonique

Ces essais démontrent donc que les compositions aqueuses selon l'invention permettent d'épaissir efficacement les formulations aqueuses acides selon l'invention et ce, tout en maintenant leur caractère limpide.

## Revendications

1. Composition aqueuse épaississante et limpide contenant, exprimé en pourcentage en poids de chaque constituant, par rapport au poids total de la formulation :
(a) de 10 % à 80 % d'au moins 2 tensioactifs non ioniques étant des alcools gras oxyalkylés,
(b) de 4 % à 40 % d'au moins un composé polyuréthanne, obtenu à partir :
d'au moins un alcool gras,
d'au moins un polyalkylène glycol, préférentiellement le polyéthylène glycol, et
d'au moins un isocyanate choisi dans le groupe consistant en le 1,4-butane diisocyanate, le 1,6-hexane diisocyanate, l'isophorone diisocyanate, le 1,3-cyclohexane diisocyanate et 1,4-cyclohexane diisocyanate, le 4,4'diisocyanatodicyclohexylmethane, le 1-methyl-2,4-diisocyanatocyclohexane et son mélange avec le 1-methyl-2,6-diisocyanatocyclohexane et leurs mélanges, et
(c) de l'eau.

2. Composition aqueuse épaississante et limpide selon la revendication 1, contenant, exprimé en pourcentage en poids de chaque constituant, par rapport au poids total de la formulation :
(a) de 20 % à 65 % d'au moins 2 tensioactifs non ioniques étant des alcools gras oxyalkylés,
(b) de 10 % à 30 % d'au moins un composé polyuréthanne et
(c) le reste d'eau.

3. Composition aqueuse épaississante et limpide selon l'une des revendications 1 ou 2, selon laquelle lesdits tensioactifs non-ioniques sont des alcools gras oxyalkylés, ayant de 3 à 40 motifs d'oxyéthylène, et ayant préférentiellement de 8 à 30 atomes de carbone, très préférentiellement de 8 à 20 atomes de carbone.

4. Composition aqueuse épaississante et limpide selon l'une des revendications 1 à 3, selon laquelle ledit alcool gras comporte de 8 à 20 atomes de carbone, préférentiellement de 10 à 18 atomes de carbone, et très préférentiellement de 10 à 16 atomes de carbone.

5. Composition aqueuse épaississante et limpide selon l'une des revendications 1 à 4, selon laquelle ledit polyalkylène glycol présente un poids moléculaire compris entre 5 000 g/mol et 20 000 g/mol, préférentiellement compris entre 8 000 g/mol et 15 000 g/mol, et très préférentiellement compris entre 8 000 g/mol et 12 000 g/mol.

6. Utilisation, pour épaissir une formulation aqueuse acide et pour conserver sa limpidité, d'une composition aqueuse épaississante et limpide contenant exprimé en pourcentage en poids de chaque constituant, par rapport au poids total de la formulation :
(a) de 10 % à 80 % d'au moins 2 tensioactifs non ioniques étant des alcools gras oxyalkylés,
(b) de 4 % à 40 % d'au moins un composé polyuréthanne, obtenu à partir :
d'au moins un alcool gras
d'au moins un polyalkylène glycol, préférentiellement le polyéthylène glycol, et
d'au moins un diisocyanate choisi dans le groupe consistant en le 1,4-butane diisocyanate, le 1,6-hexane diisocyanate, l'isophorone diisocyanate, le 1,3-cyclohexane diisocyanate et 1,4-cyclohexane diisocyanate, le 4,4'diisocyanatodicyclohexylmethane, le 1-methyl-2,4-diisocyanatocyclohexane et son mélange avec le 1-methyl-2,6-diisocyanatocyclohexane et leurs mélanges, et
(c) de l'eau.

7. Utilisation, selon la revendication 6, selon laquelle ladite composition aqueuse épaississante et limpide contient, exprimé en pourcentage en poids de chaque constituant, par rapport au poids total de la formulation :
(a) de 20 % à 65 % d'au moins 2 tensioactifs non ioniques étant des alcools gras oxyalkylés,
(b) de 10 % à 30 % d'au moins un composé polyuréthanne, et
(c) le reste d'eau.

8. Utilisation, selon l'une des revendications 6 ou 7, selon laquelle lesdits tensioactifs non-ioniques sont des alcools gras oxyalkylés, ayant de 3 à 40 motifs d'oxyéthylène, et ayant préférentiellement de 8 à 30 atomes de carbone, très préférentiellement de 8 à 20 atomes de carbone.

9. Utilisation, selon l'une des revendications 6 à 8, selon laquelle ledit alcool gras comporte de 8 à 20 atomes de carbone, préférentiellement de 10 à 18 atomes de carbone, très préférentiellement de 10 à 16 atomes de carbone.

10. Utilisation, selon l'une des revendications 6 à 9, selon laquelle ledit polyéthylène glycol présente un poids moléculaire compris entre 5 000 g/mol et 20 000 g/mol, préférentiellement compris entre 8 000 g/mol et 15 000 g/mol, et très préférentiellement compris entre 8 000 g/mol et 12 000 g/mol.

11. Utilisation, selon l'une des revendications 6 à 10, selon laquelle on met en oeuvre de 2 % à 50 %, préférentiellement de 4 % à 20 % en poids de composition aqueuse épaississante et limpide par rapport au poids total de la formulation aqueuse acide à épaissir.

12. Utilisation, selon l'une des revendications 6 à 11, selon laquelle la composition aqueuse épaississante et limpide est mise en oeuvre dans une formulation aqueuse acide contenant au moins un composé acidifiant choisi notamment parmi les acides chlorhydrique, phosphorique, sulfurique, nitrique, sulfamique, acétique, borique, citraconique, citrique, diglycolique, éthylène diamine tétraacétique, fluorhydrique, formique, glucoheptonique, glutarique, glycolique, glyoxylique, iséthionique, itaconique, lactique, maléïque, malique, méthane sulfonique, méthylsuccinique, octylique, oxalique, peracétique, propionique, saccharique, succinique, valérique, ou parmi les acides phosphoniques tels que notamment, acide 1-hydroxyéthane 1,1-diphosphonique, amino triméthylène phosphonique, éthylène diamine tétraméthyl phosphonique, diéthylène triamine pentaméthyl phosphonique, hexaméthylène diamine tétraméthyl phosphonique, hydroxyéthylamino diméthyl phosphonique, 2-phosphonobutane-1,2,4-tricarboxylique, ou les mélanges de ces acides, ou encore les mélanges de ces acides avec l'eau oxygénée.

13. Utilisation, selon l'une des revendications 6 à 12, selon laquelle la composition aqueuse épaississante et limpide est mise en oeuvre dans une formulation aqueuse acide dont le pH est inférieur à 7, préférentiellement inférieur à 6, très préférentiellement inférieur à 5, et de manière encore plus préférentielle inférieur à 4.

14. Formulation aqueuse acide épaissie et dont l'aspect est limpide, contenant une composition aqueuse épaississante selon l'une des revendications 1 à 5.

## Patentansprüche

1. Klare und verdickend wirkende wässrige Zusammensetzung, die, ausgedrückt als Gewichtsprozentsatz von jedem Bestandteil in Bezug auf das Gesamtgewicht der Formulierung, Folgendes enthält:
(a) 10% bis 80% an mindestens 2 nichtionischen Tensiden, bei denen es sich um oxyalkylierte Fettalkohole handelt,
(b) 4% bis 40% an mindestens einer Polyurethanverbindung, erhalten ausgehend von den Folgenden:
mindestens einem Fettalkohol,
mindestens einem Polyalkylenglykol, vorzugsweise Polyethylenglykol, und
mindestens einem Isocyanat, ausgewählt aus der Gruppe bestehend aus 1,4-Butandiisocyanat, 1,6-Hexandiisocyanat, Isophorondiisocyanat, 1,3-Cyclohexandiisocyanat und 1,4-Cyclohexandiisocyanat, 4,4'-Diisocyanatodicyclohexylmethan, 1-Methyl-2,4-diisocyanatocyclohexan und seiner Mischung mit 1-Methyl-2,6-diisocyanatocyclohexan und ihren Mischungen, sowie
(c) Wasser.

2. Klare und verdickend wirkende wässrige Zusammensetzung nach Anspruch 1, die, ausgedrückt als Gewichtsprozentsatz von jedem Bestandteil in Bezug auf das Gesamtgewicht der Formulierung, Folgendes enthält:
(a) 20% bis 65% an mindestens 2 nichtionischen Tensiden, bei denen es sich um oxalkylierte Fettalkohole handelt,
(b) 10% bis 30% an mindestens einer Polyurethanverbindung und
(c) als Rest Wasser.

3. Klare und verdickend wirkende wässrige Zusammensetzung nach einem der Ansprüche 1 oder 2, bei der es sich bei den nichtionischen Tensiden um oxyalkylierte Fettalkohole mit 3 bis 40 Oxyethyleneinheiten und vorzugsweise 8 bis 30 Kohlenstoffatomen, besonders bevorzugt 8 bis 20 Kohlenstoffatomen, handelt.

4. Klare und verdickend wirkende wässrige Zusammensetzung nach einem der Ansprüche 1 bis 3, wobei der Fettalkohol 8 bis 20 Kohlenstoffatome, vorzugsweise 10 bis 18 Kohlenstoffatome und besonders bevorzugt 10 bis 16 Kohlenstoffatome umfasst.

5. Klare und verdickend wirkende wässrige Zusammensetzung nach einem der Ansprüche 1 bis 4, wobei das Polyalkylenglykol ein Molekulargewicht zwischen 5000 g/mol und 20000 g/mol, vorzugsweise zwischen 8000 g/mol und 15000 g/mol und besonders bevorzugt zwischen 8000 g/mol und 12000 g/mol aufweist.

6. Verwendung einer klaren und verdickend wirkenden wässrigen Zusammensetzung, die, ausgedrückt als Gewichtsprozentsatz von jedem Bestandteil in Bezug auf das Gesamtgewicht der Formulierung, Folgendes enthält:
(a) 10% bis 80% an mindestens 2 nichtionischen Tensiden, bei denen es sich um oxyalkylierte Fettalkohole handelt,
(b) 4% bis 40% an mindestens einer Polyurethanverbindung, erhalten ausgehend von den Folgenden:
mindestens einem Fettalkohol,
mindestens einem Polyalkylenglykol, vorzugsweise Polyethylenglykol, und
mindestens einem Diisocyanat, ausgewählt aus der Gruppe bestehend aus 1,4-Butandiisocyanat, 1,6-Hexandiisocyanat, Isophorondiisocyanat, 1,3-Cyclohexandiisocyanat und 1,4-Cyclohexandiisocyanat, 4,4'-Diisocyanatodicyclohexylmethan, 1-Methyl-2,4-diisocyanatocyclohexan und seiner Mischung mit 1-Methyl-2,6-diisocyanatocyclohexan und ihren Mischungen, sowie
(c) Wasser
zum Verdicken einer sauren wässrigen Formulierung und zum Aufrechterhalten ihrer Klarheit.

7. Verwendung nach Anspruch 6, wobei die klare und verdickend wirkende wässrige Zusammensetzung ausgedrückt als Gewichtsprozentsatz von jedem Bestandteil in Bezug auf das Gesamtgewicht der Formulierung, Folgendes enthält:
(a) 20% bis 65% an mindestens 2 nichtionischen Tensiden, bei denen es sich um oxalkylierte Fettalkohole handelt,
(b) 10% bis 30% an mindestens einer Polyurethanverbindung und
(c) als Rest Wasser.

8. Verwendung nach einem der Ansprüche 6 oder 7, wobei es sich bei den nichtionischen Tensiden um oxyalkylierte Fettalkohole mit 3 bis 40 Oxyethyleneinheiten und vorzugsweise 8 bis 30 Kohlenstoffatomen, besonders bevorzugt 8 bis 20 Kohlenstoffatomen, handelt.

9. Verwendung nach einem der Ansprüche 6 bis 8, wobei der Fettalkohol 8 bis 20 Kohlenstoffatome, vorzugsweise 10 bis 18 Kohlenstoffatome und besonders bevorzugt 10 bis 16 Kohlenstoffatome umfasst.

10. Verwendung nach einem der Ansprüche 6 bis 9, wobei das Polyethylenglykol ein Molekulargewicht zwischen 5000 g/mol und 20000 g/mol und vorzugsweise zwischen 8000 g/mol und 15000 g/mol und besonders bevorzugt zwischen 8000 g/mol und 12000 g/mol aufweist.

11. Verwendung nach einem der Ansprüche 6 bis 10, wobei man 2 Gew.-% bis 50 Gew.-%, vorzugsweise 4 Gew.-% bis 20 Gew.-% an klarer und verdickend wirkender wässriger Zusammensetzung in Bezug auf das Gesamtgewicht der zu verdickenden sauren wässrigen Formulierung einsetzt.

12. Verwendung nach einem der Ansprüche 6 bis 11, wobei die klare und verdickend wirkende wässrige Zusammensetzung in einer sauren wässrigen Formulierung eingesetzt wird, die mindestens eine säuernde Verbindung, ausgewählt insbesondere aus Salzsäure, Phosphorsäure, Schwefelsäure, Salpetersäure, Sulfamidsäure, Essigsäure, Borsäure, Citraconsäure, Citronensäure, Diglykolsäure, Ethylendiamintetraessigsäure, Fluorwasserstoffsäure, Ameisensäure, Glucoheptonsäure, Glutarsäure, Glykolsäure, Glyoxylsäure, Isethionsäure, Itaconsäure, Milchsäure, Maleinsäure, Äpfelsäure, Methansulfonsäure, Methylbernsteinsäure, Octansäure, Oxalsäure, Peressigsäure, Propionsäure, Zuckersäure, Bernsteinsäure, Valeriansäure, oder unter den Phosphonsäuren wie insbesondere 1-Hydroxyethan-1,1-diphosphonsäure, Aminotrimethylenphosphonsäure,Ethylendiamintetramethylphosphonsäure,Diethylentria minpentamethylphosphonsäure,Hexamethylendiamintetramethylphosphonsäure,Hydroyethylaminodimethylphosphonsäure, 2-Phosphonobutan-1,2,4-tricarbonsäure, oder die Mischungen dieser Säuren oder auch die Mischungen dieser Säuren mit wässriger Wasserstoffperoxidlösung enthält.

13. Verwendung nach einem der Ansprüche 6 bis 12, wobei die klare und verdickend wirkende wässrige Zusammensetzung in einer sauren wässrigen Formulierung eingesetzt wird, deren pH-Wert unter 7, vorzugsweise unter 6, besonders bevorzugt unter 5 und ganz besonders bevorzugt unter 4 liegt.

14. Saure verdickte klar aussehende wässrige Formulierung, die eine verdickend wirkende wässrige Zusammensetzung nach einem der Ansprüche 1 bis 5 enthält.

## Claims

1. Thickening and clear aqueous composition containing, expressed as a percentage by weight of each constituent, relative to the total weight of the formulation:
(a) from 10% to 80% of at least 2 non-ionic surfactants being oxyalkylated fatty alcohols,
(b) from 4% to 40% of at least one polyurethane compound, obtained from:
at least one fatty alcohol,
at least one polyalkylene glycol, which is preferentially polyethylene glycol, and
of at least one isocyanate chosen from among the group consisting in the 1,4-butane di-isocyanate, 1,6-hexane diisocyanate, isophorone diisocyanate, 1,3- and 1,4- cyclohexane diisocyanate, 4,4'diisocyanatodicyclohexylmethane, 1-methyl-2,4-diisocyanatocyclohexane and its blend with the 1-methyl-2,6-diisocyanatocyclohexane and their blends, and
(c) water.

2. Thickening and clear aqueous composition according to claim 1, containing, expressed as a percentage by weight of each constituent, relative to the total weight of the formulation:
(a) from 20% to 65% of at least 2 non-ionic surfactants being oxyalkylated fatty alcohols,
(b) from 10% to 30% of at least one polyurethane compound and
(c) remaining water.

3. Thickening and clear aqueous composition according to one of claims 1 or 2, **characterised in that** said non-ionic surfactants are oxyalkylated fatty alcohols, having from 3 to 40 oxyethylene units, and having preferentially from 8 to 30 carbon atoms, very preferentially from 8 to 20 carbon atoms.

4. Thickening and clear aqueous composition according to one of claims 1 to 3, **characterised in that** said fatty alcohol has from 8 to 20 carbon atoms, preferentially from 10 to 18 carbon atoms, and very preferentially from 10 to 16 carbon atoms.

5. Thickening and clear aqueous composition according to one of claims 1 to 4, **characterised in that** said polyalkylene glycol has a molecular weight between 5,000 g/mol and 20,000 g/mol, preferentially between 8,000 g/mol and 15,000 g/mol, and very preferentially between 8,000 g/mol and 12,000 g/mol.

6. Use, to thicken an acidic aqueous formulation and to maintain its clearness, a thickening and clear aqueous composition containing, expressed as a percentage by weight of each constituent, relative to the total weight of the formulation:
(a) from 10% to 80% of at least 2 non-ionic surfactants being oxyalkylated fatty alcohols,
(b) from 4% to 40% of at least one polyurethane compound, obtained from:
at least one fatty alcohol,
at least one polyalkylene glycol, preferentially the polyethylene glycol, and at least one diisocyanate chosen from among the group consisting in the 1,4-butane diisocyanate, 1,6-hexane diisocyanate, isophorone diisocyanate, 1,3- and 1,4- cyclohexane diisocyanate, 4,4'diisocyanatodicyclohexylmethane, 1-methyl-2,4-diisocyanatocyclohexane and its blend with 1-methyl-2,6-diisocyanatocyclohexane and their blends, and
(c) water.

7. Use, according to claim 6, **characterised in that** said thickening and clear aqueous composition contains, expressed as a percentage by weight of each constituent, relative to the total weight of the formulation:
(a) from 20% to 65% of at least 2 non-ionic surfactants being oxyalkylated fatty alcohols,
(b) from 10% to 30% of at least one polyurethane compound, and
(c) remaining water.

8. Use, according to one of claims 6 or 7, **characterised in that** said non-ionic surfactants are oxyalkylated fatty alcohols, having from 3 to 40 oxyethylene units, and having preferentially from 8 to 30 carbon atoms, very preferentially from 8 to 20 carbon atoms.

9. Use, according to one of claims 6 to 8, **characterised in that** said fatty alcohol has 8 to 20 carbon atoms, preferentially from 10 to 18 carbon atoms, very preferentially from 10 to 16 carbon atoms.

10. Use, according to one of claims 6 to 9, **characterised in that** polyethylene glycol has a molecular weight between 5,000 g/mol and 20,000 g/mol, preferentially between 8,000 g/mol and 15,000 g/mol, and very preferentially between 8,000 g/mol and 12,000 g/mol.

11. Use, according to one of claims 6 to 10, **characterised in that** is implemented from 2% to 50%, preferentially from 4% to 20%, by weight of thickening and clear aqueous composition, relative to the total weight of the acidic aqueous formulation to thicken.

12. Use, according to one of claims 6 to 11, **characterised in that** the thickening and clear aqueous composition is implemented in an acidic aqueous formulation containing at least one acidifier compound chosen notably from among the hydrochloric, phosphoric, sulphuric, nitric, sulphamic, acetic, boric, citraconic, citric, diglycolic, ethylene diamine tetra-acetic, hydrofluoric, formic, glucoheptonic, glutaric, glycolic, glyoxylic, isethionic, itaconic, lactic, maleic, malic, methane sulphonic, methylsuccinic, octylic, oxalic, peracetic, propionic, saccharic, succinic, valeric acids, or from among the phosphonic acids such as notably 1-hydroxyethane 1,1-diphosphonic acid, amino trimethylene phosphonic acid, ethylene diamine tetramethyl phosphonic acid, diethylene triamine pentamethyl phosphonic acid, hexamethylene diamine tetramethyl phosphonic acid, hydroxyethylamino dimethyl phosphonic acid, 2-phosphonobutane-1,2,4-tricarboxylic acid, or the blends of these acids, or again the blends of these acids with hydrogen peroxide.

13. Use, according to one of claims 6 to 12, **characterised in that** the thickening and clear aqueous composition is implemented in an acidic aqueous formulation, the pH of which is less than 7, preferentially less than 6, very preferentially less than 5, and even more preferentially less than 4.

14. Thickened acidic aqueous formulation, the appearance of which is clear, containing a thickening aqueous composition according to one of claims 1 to 5.
